# EUROPEAN PATENT APPLICATION

(11) **EP 1 228 772 A1**
(43) Date of publication of application: **07.08.2002**
(21) Application number: 01200363.8
(22) Date of filing: 01.02.2001
(51) Int. Cl.: A61L 24/00, A61L 27/54

(54) **Novel bone cement containing bioactive agents**

(71) Applicant: AM-Pharma B.V., 2333 CD Leiden (NL)
(72) Inventor: Burger, Elisabeth Henriette, 2461 GK Ter Aar (NL)
(74) Representative: Wittop Koning, Tom Hugo

(57) **Abstract**

Described is a water based bone substitute for *in vivo* implantation, promoting bone tissue growth *in situ* comprising bone substitute material, a slow release bone growth factor and a fast release antimicrobial agent. Further, a kit and a method for the preparation of said bone substitute is disclosed.

## Description

The invention relates to a novel resorbable bone substitute for *in vivo* implantation comprising a resorbable bone substitute material, an antimicrobial agent and a bone growth factor, and to a kit and a method for the preparation thereof.

Resorbable bone substitute materials are known in the art, and are herein defined as a chemical binder, useable both in thin layers or in block form, having a cohesion providing a pressure resistance of at least 1 MPa, and providing adhesion to living bone tissue. The bone substitute material is a bone substituting implantation material, strong enough to permit loading by the patients physical movement. The bone substitute should be capable to be invaded by adjacent living bone cells and should disintegrate with time, therewith creating space, allowing viable bone tissue to grow into the said space, that is left by the resorbing material. This process is known as osteotransduction. Osteotransduction is defined as the process whereby the bone substitute material after application in a bone defect is gradually replaced by viable bone tissue. Two processes play a role in osteotransduction: first, the resorption of the substitute material and second, the biological growth of bone tissue, such that the space that was formerly occupied by the bone substitute is filled with viable bone.

In the art, solid particulate, and curable plastic resorbable bone substitute material is known. The solid particulate may comprise preformed solid particles in the form of e.g. granules or cubes, which are delivered at the location of the intended bone growth in the human or animal body. Preformed bone substitute materials are known in the art, e.g. particles of polylactic acid (PLA)or polyglycolic acid (PGA). The skilled person will be aware of other suitable preformed bone substitute materials (Tadjoedin et al., Clin. Oral Implants Res. (2000), 11, p 334-344). A bone substitute comprising preformed particles may also comprise a bio-compatible adhesive material, in order to fix the bone substitute particles at the intended location, e.g. at the site of bone damage. Such adhesive material can be in the form of a paste. The skilled person will be aware of suitable adhesive materials (Driessen et al., J. Mater. Sci. Mater in Med (1993), 4, p 503-508).

The resorbable bone substitute material may also comprise cement material of a plastic curable bone material in the form e.g. a paste, that cures *in situ* after application in the human or animal body. Examples of such bone substitute material, also known as bone cement, are calcium phosphate based cements. Such bone cement compositions comprise, after curing *in situ* at physiological conditions, a microstructure of agglutinated cement particles, wherein cavities are present between adjoining cement particles, wherein the cement particles contain micropores. Micropores are defined as pores having such dimensions, that vertebrate cells cannot pass through the said pores; the average diameter of such pores is usually below 1 µm. Such bone material is well known in the art and is e.g. described in WO96/39202 and WO99/34844, both herein incorporated by reference.

Herein, an "antimicrobial agent" is defined as any compound or preparation having a MIC (Minimal Inhibitory Concentration) of less than 10 µM. The MIC of the compound or preparation can be determined by incubating 2.5 x 10⁴ *E.coli* strain D31 bacteria overnight at 37°C in 0,25 x 100 TSB (Trypticase Solid Broth), preferably at isotonic conditions, with increasing concentrations of the compounds in e.g. microtitre plates. A compound or preparation is defined to be "antimicrobial" when the MIC of the respective compound or preparation is below 10 µM. Examples of suitable antimicrobial agents are antibiotics, antimicrobial proteins, such as lactoferrin, antimicrobial peptides (AMPs) and cholates.

A "bone growth factor" is defined herein as a compound or preparation capable of enhancing the activity of the enzyme alkaline phosphatase in pre-osteoblastic bone cells as follows (see Blom et al, J. Biomed. Mater. Res. 50,67-74,2000): pre-osteoblastic cells, released from the long bones of adult rats by collagenase treatment of the morsalized bone fragments, are grown as monolayer on tissue culture substratum. A compound or preparation is defined as bone growth factor when the level of alkaline phosphatase activity of the cells is increased by a factor of two or more at concentrations of less than 25 µg, preferably 10 µg or less, of the compound or composition per ml medium, wherein the alkaline phosphatase activity is measured 10 days after addition of the bone growth factor.

In WO 96/39202 a resorbable bone cement substitute is described, comprising biocompatible hydroxyapatite bone substitute material, and may both comprise a bone growth factor and an antibiotic agent. The bone growth factor has been incorporated in order to promote bone tissue growth, whereas the antibiotic substance is incorporated to limit bacterial growth or to avoid bacterial infection.

However, in the bone substitute of WO 96/39202, the antibiotic agent and the bone growth factor have a similar, relatively slow release profile. The slow release of both the antibiotic agent and the bone growth factor from the bone cement into the direct environment of the location where the bone cement is applied, however eliminates both of the above mentioned effects. Slow release of the antibiotic may enable any present microbe to develop resistance against the said antibiotic, resulting in severe infections that are difficult to heal and that are destroying the bone formation process, therewith interfering with the action of the bone growth factor. Said release profile may however be too fast for the growth factor, as the process of osteotransduction, whereby viable bone tissue replaces the resorbing bone substitute, takes many months, up to years (Tadjoedin E: "Histomorphometry of bone formed in the reconstructed maillary sinus" Academic Thesis, Vrije Universiteit Amsterdam, The Netherlands 2000. ISBN 90-9014251-7). It has now been found that completely different characteristics are needed for the release of the antibiotic agent and the bone growth factor, to obtain an optimal therapeutic result. In the composition as described in the art, this requirement is not met.

As with the resorbable bone substitutes according to the art, resistance against the antimicrobial agent can be developed at the location of implantation of the bone substitute, the option of incorporating antibiotic substances, as disclosed in WO 96/39202, has been regarded in the art as purely theoretic. The skilled person has been aware of the danger of development of resistant microbes, as resorbable bone substitutes comprising antibiotics are not preferred or put into practise by the skilled persons.

For the above reason, there was reluctance and a prejudice in the art against combination of an antibiotic compound and a growth factor in bone cement.

The present invention now provides a novel bone substitute having surprisingly excellent qualities; thereto the resorbable bone substitute comprises a bone substitute material, an antimicrobial agent and a bone growth factor, wherein the antimicrobial agent has a fast release profile, and the bone growth factor has a slow release profile.

Herein, "fast release profile" is defined by the release, at 37°C, of 60%, preferably 70% or more of the antimicrobial agent from the bone substitute within 48 hours, the bone substitute being in solid form and suspended in physiological phosphate buffered saline (PBS, pH 7,2), into which salt buffer the antimicrobial agent is released.

A "slow release profile" is defined by the release, at 37°C of 5% or less, preferably 2% or less, of the total amount of bone growth factor from the bone substitute, after 48 hours, the bone substitute being in solid form and suspended in physiological phosphate buffered saline, pH 7,2, into which salt buffer the bone growth factor is released.

The person skilled in the art can easily determine a release profile of any antimicrobial agent or bone growth factor: a volume of bone substitute, preferably a solid cube of 1 cm³' is suspended in physiological phosphate buffered saline for a certain time period. The concentration of the antimicrobial agent or bone growth factor in the physiological salt buffer can easily be measured at the determined time points or continuously, therewith measuring the release profile.

By providing a resorbable bone substitute comprising an antimicrobial agent having a fast release profile and a bone growth factor with a slow release profile, tremendous advantages over the resorbable bone substitutes of the art can be achieved. Having an antimicrobial agent with a fast release profile and a bone growth factor with a slow release profile in the bone substitute gives the advantage that a possible infection during or before surgery is effectively controlled and any development of resistance against the antimicrobial agent is avoided. Meanwhile, the growth factor is delivered from the bone substitute over a longer period of e.g. several months, in a very low dose; the growth factor in a bone substitute according to the invention is substantially not able to diffuse through the solid or cured bone substitute material, but is released therefrom mainly as a result of the process of resorption of the cement material. In addition, at crack formation in the cement material, which may take place *in vivo,* the bone growth factor may also be released through the surfaces of the said cracks. As this resorption of the cement material is a slow process and dependent on the invasive force of the surrounding bone tissue, the growth factor is delivered over a very long period (up to many months) in a low dose. Thus, in the bone substitute according to the invention, the antimicrobial agent is readily delivered to substantially completion within several days, whereas the growth factor shows a slow release profile. By the combination of a fast release antimicrobial agent and a slow release bone growth factor in the bone substitute according to the invention, the bone growth factor surprisingly supports the function of the antimicrobial agent by the activating effect of the bone growth factor in the host's immune system, and an accelerated recovery of the injury is observed, compared to what is expected. In addition, during the subsequent phase of osteotransduction, the slow and continuous release of the growth factor promotes the resorption of the bone substitute material and the formation of bone tissue. This leads to an improved therapeutic effect, due to a synergistic effect of the antimicrobial agent and the growth factor during the healing phase, and accelerated osteotransduction during the subsequent phase of bone substitute resorption.

The combination of a quick and completely releasing antimicrobial agent with a slow release growth factor according to the invention provides an optimal bone substitute, enabling accelerated and improved wound repair after bone surgery, without a risk of the development of resistant bacterial strains, and enabling accelerated osteotransduction thereafter.

The growth factor is preferably uncharged as physiological pH. Such a growth factor is substantially not released from the micropores of the bone substitute material by diffusion processes, but in vast majority upon desintegration of the bone substitute, as indicate above, therewith ensuring the required slow release profile.

In a preferred embodiment, the bone growth factor is chosen from the TGFβ superfamily. The term "TGFβ superfamily" is known in the art (Kingsley D.M. Genes and Development 8: 133-146, 1994). Among others and in addition to TGFβ, growth and differentiation factors (GDFs), and bone morphogenetic proteins (BMPs) belong to the said superfamily (Massague J., Animal Review of Cell Biology, 6: 597-641, 1990). However, a vast number of bone growth factors are known in the art, which can effectively and advantageously be used in the bone substitutes according to the present invention. In this respect, the bone growth factors, listed in WO96/39202 may be mentioned.

The bone substitute according to the invention preferably comprises 0.1-30 µg bone growth factor per cm³ bone substitute material.

The growth factor is preferably associated with a carrier, preferably a protein, such as, preferably, blood serum proteins, e.g. bovine or human serum albumin. As the amount of bone growth factor in the bone substitute of the invention is relatively low, aspecific binding interactions interfering in the release profile of the bone growth factor is to be avoided. The carrier protein may avoid aspecific binding of the growth factor to e.g. glass and plasticware during the preparation, and, further, it is thought that it may help to keep the growth factor on its place in the bone cement and prevents diffusion therefrom within the bone substitute, e.g. cured cement, thereby securing the slow release of the growth factor from the cement. Also, other biocompatible carrier materials are to be contemplated, such as whole human plasma and human collagen. The carrier should preferably be present in excess over the bone growth factor, therfore per cm³ bone substitute material, 0,1-4 mg carrier, or more, is preferably present in the bone substitute material.

As indicated above, the antibiotic compounds that have been proposed for incorporation in resorbable bone substitute material show a release profile that is too slow.

It has now surprisingly been found that incorporation of both a growth factor and an antimicrobial peptide (AMP) in a bone substitute provides an excellent bone substitute having the above discussed unexpected synergistic qualities. The present inventors have found that incorporation of small antimicrobial peptides have the capacity to show a fast release profile from a wide variety of bone substitutes, such as solid bone substitute particles such as PLA or cured calcium-phosphate based bone cements and, in particular, curable calcium phosphate-based bone cements.

Antimicrobial peptides are known in the art. AMPs are natural occurring antibiotic molecules, found, among others, in saliva, such as histatins and defensins. Histatins have strong antibacterial and antifungal properties and form part of the natural defence system against infections in mammals and other vertebrates (see e.g. Oppenheim et al, J. Biol. Chem 263 (2000) pp 7472-7477 and Selstedt et al, J. Clin. Invest. 76 (1985) pp 1436-1439). In previous work, it has been found that synthetic and truncated derivatives of histatins exhibit a wide spectrum of antibacterial activity, including against the methycilline resistant *Staphylococcus aureus,* a bacterium that is very difficult to control (Lyaruu et al J. Dent. Res. 79, IADR Abstracts No. 669, p. 227 (2000)).

It has surprisingly been found that, compared with their native AMP counterparts, synthetic and truncated derivatives of AMPs having aliphatic properties, especially derived from histatins, show an improved fast release profile, and, even more surprisingly, retain their substantially full antimicrobial activity at isotonic conditions, implicating that these derivatives can advantageously be used within tissues, while native histatins in saliva usually have their optimal activity under hypotonic conditions of e.g. the oral cavity. Such histatin derivatives are described in WO00/367678 and in WO00/01427, both being herein incorporated by reference. Below, the terms "antimicrobial peptide",or "AMP" also encompass the above-mentioned synthetic and truncated such derivatives.

A preferred antimicrobial peptide for incorporation in the bone substitute according to the invention is therefore an antimicrobial peptide of 10 to 25 amino acids in length, comprising a domain of at least 10 amino acids, consisting of two sterically oppositely arranged subdomains (also referred to as "terminal halves"), wherein the majority of the amino acids of the first domain being positively charged at physiological pH and the majority of the amino acids of the second subdomain being uncharged at physiological pH.

AMPs comprising such a domain have now been shown to have a surprisingly fast diffusion profile from the bone substitute and having high antibiotic activity at isotonic conditions.

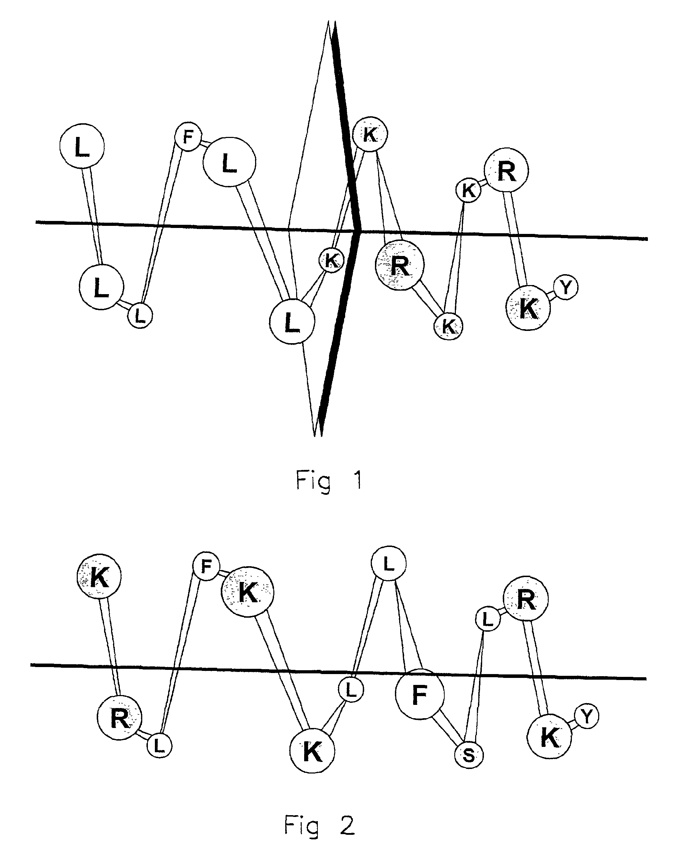

The antibiotic activity is thought to be caused by the aliphatic properties of the AMP: because of the aliphaticity, the AMP may cause penetration of the AMPs into the bacterial membrane and eventually to leakage of the bacterial cells. For the aliphatic properties, and therefore for antimicrobial activity, it is important that in the domain, a charge difference between both subdomains is present at physiological pH. Hereto, the subdomains are arranged sterically opposite to one another; said arrangement of the subdomains can be arranged in several ways, e.g. when the tertiary strucure of the domain comprises an α-helix structure, the first subdomain may be located at the amino terminal half of the domain, and the second subdomain at the carboxy terminal half thereof, or vice versa. This arrangement may be regarded as "terminal" aliphaticity. An example of an AMP having axial aliphaticity is shown in figure 1. Said peptide has the amino acid sequence according to Seq. ID no. 4. The subdomains may also be axially arranged in the domain, i.e. one side of the helix, in axial view, is mainly positively charged (constituting the first subdomain), and the other, opposite side of the helix in axial view, is mainly uncharged (constituting the second subdomain). This arrangement may be regarded as "axial" aliphaticity. An example of an AMP having axial aliphaticity is shown in figure 2. Said peptide has the amino acid sequence according to Seq. ID No. 3. In a further embodiment, the domain may comprise a β-sheet structure, comprising the two subdomains in the above described axial or terminal arrangement. On primary structure level (i.e. on the level of the plain amino acid sequence), terminal arrangement of the subdomains implies that both subdomains are each formed by a continuous stretch of amino acids; preferably, both subdomains are adjacent to one another; however both subdomains may be separated from one another by e.g. additional amino acids, as long as the aliphatic character, and therewith the antimicrobial activity as well as the fast release profile is maintained. In case of axial arrangement, the amino acids of the subdomains are not defined by a continuous stretch of amino acids on the primary structure level, but the subdomain is merely defined by the tertiary structure thereof.

In another preferred embodiment, a lactoferrin derived peptide with antimicrobial properties is incorporated in the bone cement. In particular, the lactoferrin derived peptide comprises the amino acids 11-30 of the natural lactoferrin (Seq. ID No. 5), which peptide shows good antimicrobial activity against numerous bacteria and fungi causing MRSA-infections. In a very special embodiment, the bone cement according to the invention comprises a peptide comprising the amino acid sequence, corresponding with the first eleven amino terminal amino acids of the natural lactoferrin (Seq. ID No. 6), showing an even improved antimicrobial activity against most bacteria and fungi causing MRSA-infections. Preferably, the peptide has a length of 25 amino acids or less and preferably consists of the above-mentioned amino acid sequences (Seq. ID No. 5 or 6).

Cystatine derived peptides having antimicrobial peptides can also advantageously be incorporated in the bone cement according to the present invention. Cystatines are natural proteins, present in human, animal and plants and are capable of specifically inhibiting the class of cysteine proteinases (Blankenvoorde et al, Biol. Chem. 377, 847-850 (1996)). Inflammatory processes, caused by e.g. porfiromonas gingivalis, can effectively be inhibited by cystatines and by cystatine derived peptides. Such peptides, showing a fast release profile, are effective in the bone cement according to the present invention. Advantageously, a cystatine derived peptide comprising the fourteen amino terminal amino acids of cystatine (Seq. ID No. 7) is incorporated in the bone cement.

For optimal antibiotic activity of the AMP to be incorporated in the bone substitute according to the invention, it is very advantageous when the charge differences of both subdomains are as large as possible. Therefore, the domain is preferably free of any negatively charged amino acids.

Among the best performing peptides for incorporation in the bone cement composition according to the invention, the domain of the AMP or AMP derivative preferably contains one of the following sequences:

KRKFHEKHHSHRGY (Seq. ID No. 1)

KRLFKKLKFSLRKY (Seq. ID No. 2)

KRLFKKLLFSLRKY (Seq. ID No. 3)

LLLFLLKKRKKRKY (Seq. ID No. 4)

FKCRRWQWRMKKLG (Seq. ID No. 5)

GRRRRSVQWCA    (Seq. ID No. 6)

SSSKEENRIIPGGI (Seq. ID No. 7)

Seq. ID No. 1 reflects the natural carboxyterminal of the natural human histatin 5, also referred to as DH5 (Helmenhorst et al, 1997, Biochem. J. 326, pp. 39-45). i.e. a truncated histatin derivative, whereas Seq. ID Nos. 2, 3 and 4 reflect synthetic derivatives, based on Seq. ID No. 1. The synthetic derivatives, especially comprising Seq. ID No. 4 or Seq. ID No. 6, are preferred, for a high antibiotic activity and very suitable fast release profile that can be obtained.

The above-mentioned domain of the AMP may have a length of 25 amino acids, but is preferably less. Preferably, the AMP contains less than 15 amino acids, more preferably, the domain makes up the entire peptide. A smaller peptide generally will show a faster release profile from the bone substitute compared to a larger peptide. Therefore, if the said release should be attenuated somewhat, it can be contemplated to use longer AMPs. The domain may be flanked by other amino acids on either or both sides thereof. It is preferred to use AMPs that comprise two domains that may be coupled to one another head to head, tail to tail or head to tail. In the art, methods are known for the preparation of such confirmations; reference is made to WO99/37678. It is also possible to attenuate the delivery of antimicrobial agents, such as AMPs from the bone cement by e.g. encapsulating the AMPs in suitable polymers, such as polyacrylamide or xanthan gum, guar gum, carboxy methylcellulose. Such measures are known in the art. The skilled person will know how to encapsulate the antimicrobial agent for the desired aim.

Preformed solid particles may also advantageously be used in the resorbable bone substitute according to the invention.

The preformed particles may be in the form of cubes, spheres and the like, and can be used to fill a volume in an injured bone. In another embodiment, the particle has a preshaped form of a bone or part thereof, to replace such a bone or bone part. Such preshaped forms are e.g. suitable to be used in orthopedic and spine surgery, and may have the form of e.g. carpal, metacarpal, mandible, costa or vertabra of part thereof.

The preformed particles may comprise any known suitable biocompatible resorbable material, such as e.g. PLA, PGA, or (ex vivo) cured calcium phosphate-based bone cements.

In case of preformed bone substitute particles, the bone growth factor may be incorporated in the micropores of the said particles, whereas the antibiotic may be coated onto the said particles, enabling a fast release of the antibiotic agent. For such a coating, it is also possible to use the above described encapsulated antibiotic agents therein. When a separate adhesive is used for binding the particles to one another and/or to the bone material at the location of bone injury in the patient, the antimicrobial agent can be incorporated in the adhesive material. The antimicrobial agent may also be incorporated in the particles, as long as a fast release profile is obtained.

In a preferred embodiment, the bone substitute comprises curable bone cement material, as it has been found that excellent results can be obtained with such material. Preferably, the cement comprises calcium phosphate, being chosen from the group, consisting of dicalcium phosphate, tricalcium phosphate, tetracalcium phosphate hydroxylapatite or a mixture of two or more thereof. It has been found that AMPs, especially the above-mentioned derivatives show the required slow release profile when incorporated in calcium phosphate-based curable bone cements. The skilled person will know the proper ratios of the abovementioned compounds for the envisaged application.

The bone substitute may also advantageously be accomodated in an outer structure of solid biocompatible resorbable material, such as PLA or PGA. The outer structure may be in the form of a cube, wherein the bone substitute according to the invention is incorporated. Such cubes may be closed or open, e.g. only consisting of the cube ribbons. Other forms of the outer structure are also possible, such as e.g. tetra- or oktaeder forms, or forms corresponding to the shape of a bone or part thereof, as is described above. Such structures wherein bone substitute material is accomodated are known in the art as so-called "cages" and are e.g. used in spine surgery.

Both growth factor and antimicrobial agent are preferably evenly distributed throughout the bone substitute; in case of a bone substitute comprising preformed solid particles having a coat or is combined with an adhesive, the growth factor is preferably evenly distributed throughout the said solid particles, whereas the antimicrobial agent is in that case evenly distributed throughout the coat or adhesive, respectively.

In the art, bone substitutes are prepared by mixing an aqueous phase with dry cement material, to obtain a bone substitute mass in the form of e.g. a moldable paste, that may be cured before or after application in a patient's body.

Addition of bone growth factor is however problematic; as relatively small amounts of bone growth factor are required (0,1-10 µg/g cement), a carrier, preferably a carrier protein as described above, is found to be needed to avoid loss of growth factor due to aspecific binding, as discussed above. Therefore, it is important to add the bone growth factor to an aqueous medium that already comprises carrier material. However, if the bone growth factor is added to the complete required volume for the bone substitute preparation, the corresponding amount of carrier has been found to interfere with the curing process of the bone substitute material. Furthermore, the bone growth factor may be supplied in a buffer that is not compatible with the bone cement powder material. For example TGFβ is stably stored in a buffer comprising 4mM HCl. It should preferably be avoided that the liquid phase added to the cement powder comprises such amount of HCl. Accordingly to the invention, the bone growth factor is therefore preferably provided in a relatively small first volume in order to limit the amount of carrier and incompatible ions. A second volume is either added to the said first volume, or both volumes are added to the cement material separately.

The invention therefore also relates to a method for the preparation of a resorbable bone substitute according to the invention, comprising the step of mixing a liquid aqueous component and a dry component comprising the bone cement powder material, wherein the preparation of the liquid component comprises the steps of:
a) providing a first volume of a first aqueous medium, prepared by adding the bone growth factor in the said medium, comprising carrier protein,
b) providing a second volume of a second aqueous medium.

It has now been found that when the bone growth factor is mixed with a first volume of a first aqueous medium that already comprises the carrier, the amount of carrier is sufficient to prevent loss of activity of the bone growth factor, but does not lead to interference with the curing process of the bone substitute. The second volume of the second medium supplements the required volume needed for the preparation of the bone substitute. Both first and second media can be mixed together before mixing with the dry component; in case, the first and second media have different volumes, it is preferred to add the larger volume first.

The volume ratio between the first and second aqueous medium is preferably 1:1 to 1:10.

As outlined above, the amount of carrier is preferably in excess over the amount of bone growth factor. An excess of a factor 100-1000 is effective, although the skilled person will be capable of finding a proper excess factor.

In a preferred embodiment of the invention, the antimicrobial agent is added to the second aqueous medium or to a mixture of both first and second media. In this case, the antimicrobial agent is present in the aqueous component.

In another embodiment of the method according to the invention, the antimicrobial agent is incorporated in the dry component in dry form. In that case, the AMP is preferably freeze dried and mixed with the cement formulation (e.g. BiobonR from ETEX Corp. Cambridge MA, USA) to form the dry component.

The bone substitute preferably comprises 0,1-10 mg the antimicrobial agent and 0,1-10 µg bone growth factor per g dry bone cement powder.

As outlined above, the bone growth factor preferably comprises TGFβ, whereas the microbial agent preferably comprises an aliphatic antimicrobial peptide as defined above.

The invention also relates to a kit for the preparation of a resorbable bone substitute according to the invention, comprising:
- a liquid aqueous component comprising bone growth factor and carrier protein, and
- a solid component comprising bone cement material,
the antimicrobial agent being incorporated in the liquid or solid component or both, preferably in the solid component.

With such a kit, the bone substitute can easily be prepared by mixing both components.

Preferably, the liquid component of the kit comprises, per g powder bone cement material in the solid component:
- 0,2-20 µg bone growth factor, preferably TGFβ,
- 0,2-8 mg carrier protein, preferably human serum albumin,
- 0-20 mg antimicrobial agent, preferably an aliphatic antimicrobial peptide as defined above.

The antimicrobial agent is incorporated in either one of the dry or liquid component, or both, and the total amount thereof is in this embodiment 0,2-20 mg.

The aqueous component of the kit may also be divided in two subcomponents, of which the first subcomponent comprises the bone growth factor and the carrier in a first aqueous medium, whereas the second subcomponent comprises a second aqueous medium, free of bone growth factor and carrier. As it outlined above, it is preferred to limit the amount of carrier in the bone substitute material. Also, the amount of medium, necessary for stable storage of the bone growth factor should be limited in the bone substitute material, as this medium may interfere with proper curing of the bone substitute material. The first subcomponent may therefore comprise a relatively high amount of carrier and the incompatible first medium, but these components are diluted to below critical amounts by adding the second aqueous medium.

The invention will now be further illustrated with the following not limiting examples.

### EXAMPLE 1

### Curable bone substitute comprising TGFβ and an antimicrobial peptide

One mg anti-microbial peptide DHVAR-5 (LLLFLLKKRKKRKY, seq id no 4) is mixed with 1 g BiobonR cement powder (ETEX Corp. Cambridge, MA, USA) The growth factor Transforming Growth Factor-beta (TGFβ) is suspended in a solution of 0.2% Serum Albumin in 4mM HCl, at 1 µg TGFβ per ml solution, forming the first aqueous medium.
This suspension is mixed with an equal volume of a second aqueous medium, comprising 4% Na₂PO₄. Both first and second media are combined and mixed.
1 Gram of the dry component, DHVAR-5 enriched cement powder, is mixed with 0.8ml of the liquid component, TGFβ enriched cement liquid. This gives a moldable paste that hardens within 5 minutes.
The bone substitute obtained comprised 1 mg antimicrobial peptide and 0.4 µg TGFβ per g cement.

### EXAMPLE 2

### Release kinetics of the bone substitute

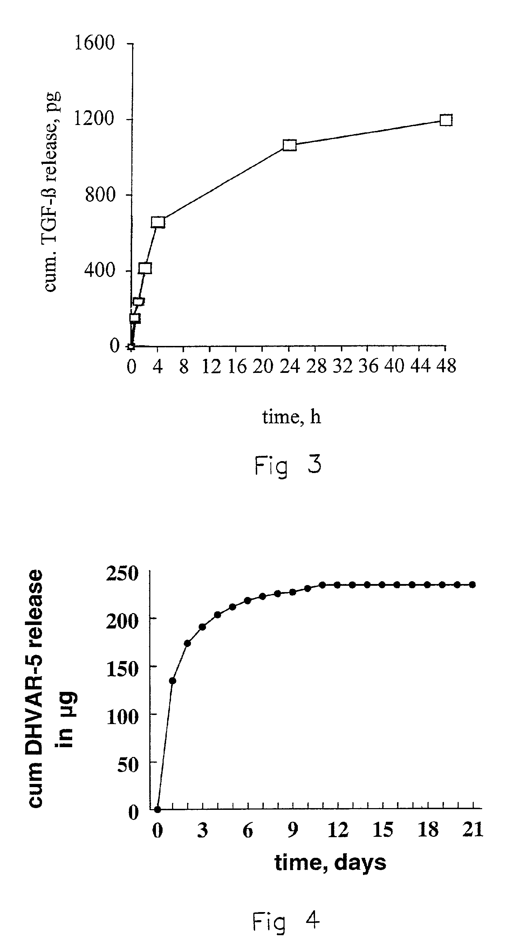

The release kinetics of the bone growth factor and of the antimicrobial peptide from bone substitute have been determined as follows:
In the release experiments the cement powder (40 mg) was mixed on a glass plate with TGFβ-containing liquid (13.3 µl), giving a paste of 53 mg cement with 133 ng TGFβ and 250 µg DHVAR 5. After 1 min of mixing the cement paste was applied into a Teflon mould with a diameter of 5 mm and measuring 1 mm in height. After 2 min the cement pellets were removed from the mould and added to the wells of 12-well culture plates (Costar, Cambridge, MA, USA). Complete culture medium (Dulbecco's modified Eagle's medium (DMEM, Gibco-BRL Life Technologies Ltd. Paisley, UK) with 10% fetal bovine serum (FBS; Gibco) was added at 1 ml per well, to wells containing a TGFβ DHVAR5 enriched cement pellet as described above. The multiwell plates were incubated at 37°C in 5% CO₂ and 95% humidity. Culture medium was renewed completely at suitable time intervals (0.5, 1, 2, 4, 24, 48 hr and 1, 2, 4 and 8 weeks), and the medium samples were stored at - 20°C until used for TGFβ and DHVAR 5 determination. TGFβ was determined in the collected culture medium samples using a commercially available enzyme-linked immunosorbant assay (ELISA) (Promega, Madison, WI, USA). A few cement pellets were crushed in the well with a mortar at 48 hr, after removal of the medium. The fragments were incubated in fresh medium for 1 hr; medium was then removed and stored for DHVAR5 and TGFβ determination. The amount of DHVAR5 released was determined by subjecting medium samples to capillary zone electrophoresis. Biological activity against *S*. *aureus* was confirmed in a standard killing assay. TGFβ was assayed by standard immunological analysis and by examination of the potency to promote cell growth as described above. The release of TGFβ from a cement pellet that incorporated TGFβ and DHVAR5 (133 ng TGFβ and 250 µg DHVAR 5 in 53 mg cement) at the time of setting of the cement, is shown in figures 3 and 4 respectively. A rapid TGFβ release was found in the first 4 hr followed by a much slower release. The cumulative release found after 4 hr was 0.5% of the original amount of TGFβ mixed in the cement and increased to 1% after 48 hr. Release of TGFβ continued slowly hereafter, with maximal 0.1% released during the next 8 weeks (data not shown). The pellets that were fragmented at 48 hr released approximately 0.5% TGFβ within 1 hr after fragmentation (data not shown), indicating that the release is surface dependent. DHVAR 5 showed a release of 70% within 48 hours; after 4 days, more than 80% of the antimicrobial peptide was released from the cement material. Similar results were obtained with peptides of Seq. ID Nos. 1-3, 5-7. In a similar test, the antibiotic gentamicin showed incomplete and slow release kinetics.

### Example 3

### Antibacterial activity and osteotransductive activity of the bone substitute in vivo

This experiment demonstrates the improved therapeutic effect of the combination of short-term released antimicrobial agent and long-term released growth factor in a resorbing bone cement.
(for details of the experimental techniques, see the PhD thesis of J.P.Eerenberg, " Taurolin in the treatment of experimental post-traumatic osteomyelitis", Vrije Universiteit, Amsterdam, the Netherlands 1996).
The model is a Foreign Body Bone Infection Model in rabbits. Under general anaesthesia, a steel thread is inserted in the rabbit femur via a hole drilled in the trochanter major. Then, a second hole is drilled in the middle of the diaphysis, and an inoculum of methicillin resistant *Staphylococcus aureus* is introduced in the marrow cavity. This procedure mimicks a post-traumatic, foreign body-associated bone infection. Subsequently, self setting bone cement is used to plug the mid-diapyseal hole. As resorbing, self setting bone cement the bone substitute according to example 1 was used; as control, a corresponding cement without DHVAR-5 and TGFβ was used. Further, corresponding cement formulations with either DHVAR-5 or TGFβ were used. The experiment was done with six groups, each consisting of 8 rabbits. Thus, the following groups were used:
Group 1, no additions (control group)
Group 2, DHVAR-5 only
Group 3, TGFβ only
Group 4, DHVAR-5 plus TGFβ
Group 5, DHVAR-5 only,
Group 6, DHVAR-5 plus TGFβ
After 4 weeks groups 1, 2, 3 and 4 are killed, and the femora were analysed for osteomyelitis using X-rays, bacterial cultures of bone and soft tissues, and histology. Ninety % of the animals of group 1 showed an osteomyelitis infection, as well as 80% of group 3. None of the animals of groups 2 and 4 has osteomyelitis, but the inflammatory reaction has subsided earlier in group 4 than 2.
After 8 weeks groups 5 and 6 are killed. None of the animals show osteomyelitis, but in group 5 little bone has formed in the diaphyseal hole which still contains much cement. In group 6, osteomyelitis is absent while the amount of bone filling the hole is larger and the amount of remaining cement is smaller then in group 5, showing that osteotransduction was accelerated.
Together, the data show that the combined enrichment has prevented the development of osteomyelitis and accelerated and improved osteotransduction.

## Claims

1. Resorbable bone substitute for *in vivo* implantation, comprising bone cement material, an antimicrobial agent and a bone growth factor, wherein
- the antimicrobial agent has a fast release profile, and
- the bone growth factor has a slow release profile.

2. Resorbable bone substitute according to claim 1, wherein the growth factor is uncharged at physiological pH.

3. Resorbable bone substitute according to any of the preceding claims, wherein the bone growth factor is chosen from the TGFβ superfamily, the bone growth factor preferably comprising TGFβ.

4. Resorbable bone substitute according to any of the preceding claims, comprising 0,1-30 µg bone growth factor per cm³ bone substitute material.

5. Resorbable bone substitute according to any of the preceding claims, comprising a carrier protein, preferably chosen from blood serum proteins, preferably serum albumin, preferably from mammalian, most preferably from human origin.

6. Resorbable bone substitute according to claim 5, comprising per cm³ bone substitute material 0,1-4 mg carrier, preferably comprising carrier protein.

7. Resorbable bone substitute according to any of the preceding claims, wherein the antimicrobial agent comprises an antimicrobial peptide being 10 to 25 amino acids in length, comprising a domain of at least 10 amino acids, consisting of two sterically oppositely arranged subdomains, wherein the majority of the amino acids of the first subdomain being positively charged at physiological pH, and the majority of the amino acids of the second subdomain being uncharged at physiological pH.

8. Resorbable bone substitute according to claim 7, the domain of the antimicrobial peptide being free of negatively charged amino acids at physiological pH.

9. Resorbable bone substitute according to claim 7 or 8, wherein the domain of the antimicrobial peptide being chosen from the following sequences: KRKFHEKHHSHRGY (Seq. ID No. 1) KRLFKKLKFSLRKY (Seq. ID No. 2) KRLFKKLLFSLRKY (Seq. ID No. 3) LLLFLLKKRKKRKY (Seq. ID No. 4) FKCRRWQWRMKKLG (Seq. ID No. 5) GRRRRSVQWCA    (Seq. ID No. 6) SSSKEENRIIPGGI (Seq. ID No. 7), the domain preferably being LLLFLLKKRKKRKY (Seq. ID No. 4) or GRRRRSVQWCA (Seq. ID No. 6),

10. Resorbable bone substitute according to any of the preceding claims, comprising curable bone cement material.

11. Resorbable bone substitute according to any of the preceding claims 1-10, comprising coated solid bone cement particles of biocompatible resorbable material, wherein the bone growth factor is incorporated in the particles, and the coat comprises the antimicrobial agent.

12. Resorbable bone substitute according to any of the preceding claims, comprising per g bone cement material:
0,1-10 µg growth factor,
0,1-10 mg antimicrobial peptide

13. Method for the preparation of a resorbable bone substitute according to any of the preceding claims, comprising the step of mixing a liquid aqueous component and a dry component comprising the bone cement powder material, wherein the preparation of the liquid component comprises the steps of:
a) providing a first volume of a first aqueous medium, prepared by adding the bone growth factor in the said medium, comprising carrier protein,
b) providing a second volume of a second aqueous medium.

14. Method according to claim 13, wherein the volume ratio between the first aqueous medium and the second aqueous medium is 1:1 - 1:10.

15. Method according to claim 13 or 14, wherein the bone growth factor comprises TGFβ.

16. Method according to any of the claims 13-15, wherein the antimicrobial agent comprises an antimicrobial peptide as defined in claims 7-9.

17. Kit for the preparation of a resorbable bone substitute according to any of the preceding claims, comprising:
- a liquid aqueous component comprising bone growth factor and carrier protein, and
- a solid component comprising powder bone cement material,
the antimicrobial agent being incorporated in the liquid or solid component or both, preferably in the solid component.

18. Kit according to claim 17, wherein the liquid component comprises, per g powder bone cement material:
- 0,2-20 µg bone growth factor, preferably TGFβ,
- 0,2-8 mg carrier protein, preferably human serum albumin,
- 0-20 mg antimicrobial agent, preferably an antimicrobial peptide as defined in claims 7-9, and
1 ml water or aqueous medium, wherein the amount of antimicrobial agent in the kit is at least 0,2 mg per g powder bone cement material.

19. Kit according to claim 17 or 18, wherein the aqueous component comprises two aqueous subcomponents, the first subcomponent comprising the bone growth factor and the carrier in a first aqueous medium, the second subcomponent comprising a second aqueous medium, free of bone growth factor and carrier.


SEQUENCE LISTING

<110> AM Pharma BV

<120> Bone cement

<130> Bone Cement - AM Pharma

<140>
<141>

<160> 7

<170> PatentIn Ver. 2.1

<210> 1
<211> 14
<212> PRT
<213> Homo sapiens

<400> 1
Lys Arg Lys Phe His Glu Lys His His Ser His Arg Gly Tyr
  1               5                  10

<210> 2
<211> 14
<212> PRT
<213> Artificial Sequence

<220>
<223> Description of Artificial Sequence:Peptide
      analogue of C-terminus of human histatin DH5

<400> 2
Lys Arg Leu Phe Lys Lys Leu Lys Phe Ser Leu Arg Lys Tyr
  1               5                  10

<210> 3
<211> 14
<212> PRT
<213> Artificial Sequence

<220>
<223> Description of Artificial Sequence:Peptide
      analogue of C-terminus of human histatin DH5

<400> 3
Lys Arg Leu Phe Lys Lys Leu Leu Phe Ser Leu Arg Lys Tyr
1               5                   10

<210> 4
<211> 14
<212> PRT
<213> Artificial Sequence

<220>
<223> Description of Artificial Sequence:Peptide
      analogue of C-terminus of human histatin DH5

<400> 4
Leu Leu Leu Phe Leu Leu Lys Lys Arg Lys Lys Arg Lys Tyr
1               5                   10

<210> 5
<211> 14
<212> PRT
<213> Homo sapiens

<400> 5
Phe Lys Cys Arg Arg Trp Gln Trp Arg Met Lys Lys Leu Gly
1               5                   10

<210> 6
<211> 11
<212> PRT
<213> Homo sapiens

<400> 6
Gly Arg Arg Arg Arg Ser Val Gln Trp Cys Ala
1               5                   10

<210> 7
<211> 14
<212> PRT
<213> Homo sapiens

<400> 7
Ser Ser Ser Lys Glu Glu Asn Arg Ile Ile Pro Gly Gly Ile
1               5                   10